# EUROPEAN PATENT APPLICATION

(11) **EP 3 881 853 A1**
(43) Date of publication of application: **22.09.2021**
(21) Application number: 20164727.8
(22) Date of filing: 20.03.2020
(51) Int. Cl.: A61K 35/407, A61P 37/00, A61P 31/12, A61P 1/00, A61P 11/00, C12N 5/071

(54) **HUMAN ALLOGENEIC LIVER-DERIVED PROGENITOR CELLS FOR USE IN THE TREATMENT OF INFLAMMATORY AND INFECTIOUS LUNG DISEASES AND SYSTEMIC INFLAMMATION**

(71) Applicant: PROMETHERA THERAPEUTICS SA, 1435 Mont-Saint-Guibert (BE)
(72) Inventor: TCHELINGERIAN, Jean-Leon, 1435 Mont-Saint-Guibert (BE); OTTEN, Luc, 1435 Mont-Saint-Guibert (BE); NAJIMI, Mustapha, 1435 Mont-Saint-Guibert (BE)
(74) Representative: Brantsandpatents bvba

(57) **Abstract**

The current invention relates to human allogeneic liver-derived progenitor cells (HALPCs), a composition comprising a therapeutically effective amount of said human allogeneic liver-derived progenitor cells or conditioned medium obtainable by culturing said cells in a cell medium for use in the (symptomatic) treatment of inflammatory lung diseases, infectious lung diseases and/or systemic inflammation.

## Description

### FIELD OF THE INVENTION

The present invention relates to human allogeneic liver-derived progenitor cells that are generated using primary liver cells for use in the treatment or as symptomatic treatment of inflammatory respiratory illness or inflammatory or infectious lung diseases and/or systemic inflammation in a subject.

### BACKGROUND

Stem and progenitor cells have been extensively used in cell therapy, which includes a plethora of preclinical research investigations as well as a significant number of clinical trials. MSCs for instance have been described for use in the battle of inflammatory pulmonary diseases. Acute respiratory distress syndrome (ARDS) for instance, a clinical complication of acute lung injury (ALI), is a devastating condition characterised by a dysfunction of the lung epithelial barrier and the capillary endothelium, diffuse alveolar damage and interstitial oedema, leading to rapidly progressive acute respiratory failure. ARDS can be caused by various clinical disorders, most commonly bacterial or viral pneumonia, sepsis, major trauma or aspiration. MSCs have proven to be efficacious in various animal models and could provide for a cell-based therapy. WO2015016761 for instance describes the use of MSCs in the treatment of inflammatory pulmonary diseases such as ARDS.

Infections of bacterial and viral origin may lead to severe respiratory complications such as pneumonia, acute pulmonary disease or even systemic inflammation. In the past it has been reported that stem cell or progenitor cell therapy may be used in the treatment of infection diseases and their complications. Shetty, 2020 and Leng et al., 2020 for instance report the use of mesenchymal stem cells (MSCs) in patients being infected with coronavirus disease 2019 (COVID-19), also known as SARS-Cov-2. This study demonstrated that intravenous infusion of MSCs is a safe and effective approach for treating patients with COVID-19 pneumonia, including elderly patients displaying severe pneumonia. This MSC therapy is believed to inhibit the overactivation of the immune system and promoting endogenous repair by improving the lung microenvironment after viral infection. Chan et al., 2016 describes the use of human mesenchymal stromal cells to reduce influenza A H5N1-associated acute lung injury.

While MSCs are thus reported to be useful against respiratory illness caused by infection, there remains a need in the art for further possible cellular treatment options, especially those that may help in battling further subsequent symptoms of the infection, such as gastrointestinal symptoms (next to the respiratory symptoms).

WO 2016/030525 discloses specific cell culture conditions allowing the obtention of human adult liver-derived progenitor cells (HALPC) with specific expression profile and improved biological features. Such conditions can be used for producing either cell-based pharmaceutical compositions that can be administered for the treatment of liver diseases, or metabolically and hepato-active cells that can be used for characterizing the efficacy, metabolism, and/or toxicity of a compound.

Sokal et al., 2017 describes the biodistribution of HALPCs after infusion of the cells in the body. Cells are shown to arise in the lungs, from where they then migrate to the liver. The ability of these cells to appear in the lungs, while subsequently migrating to other parts of the body, such as the gastrointestinal tract, provides enormous possibilities to use said cells in the treatment of pulmonary and gastrointestinal symptoms linked to infections.

### SUMMARY OF THE INVENTION

The present invention and embodiments thereof describes for the first time the use of allogeneic liver progenitor cells that are generated using primary liver cells for use in the treatment or as symptomatic treatment of inflammatory respiratory illness or lung diseases and/or systemic inflammation in a subject, caused by an infection. More in particular, the current invention describes a use according to claim 1 and dependent claims.

It has now been unexpectedly found that said cells provide a cell therapy for respiratory illnesses, gastro-intestinal symptoms and systemic inflammation, linked to infections such as viral infections.

### DETAILED DESCRIPTION OF THE INVENTION

The present invention describes for the first time the use of human allogeneic liver-derived progenitor cells (HALPCs), a composition comprising a therapeutically effective amount of said human allogeneic liver-derived progenitor cells or a conditioned medium obtained from culturing said HALPCs in the (symptomatic) treatment of inflammatory lung diseases, infectious lung diseases as well as systemic inflammation in a subject.

In another or further embodiment, the present invention equally concerns human allogeneic liver-derived progenitor cells (HALPCs) or a composition comprising a therapeutically effective amount of said human allogeneic liver-derived progenitor cells for use in reducing or preventing gastro-intestinal symptoms in a subject caused by an infection.

### DEFINITIONS

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention. The terms or definitions used herein are provided solely to aid in the understanding of the invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The recitation of numerical ranges by endpoints includes all numbers and fractions subsumed within that range, as well as the recited endpoints.

The expression "% by weight", "weight percent", "%wt" or "wt%", here and throughout the description unless otherwise defined, refers to the relative weight of the respective component based on the overall weight of the formulation.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

The term "liver progenitor cell" refers to an unspecialized and proliferation-competent cell which is produced by culturing cells that are isolated from liver or part thereof and which or the progeny of which can give rise to at least one relatively more specialized cell type. A liver progenitor cell give rise to descendants that can differentiate along one or more lineages to produce increasingly more specialized cells (but preferably hepatocytes or hepato-active cells), wherein such descendants may themselves be progenitor cells, or even to produce terminally differentiated liver cells (e.g. fully specialized cells, in particular cells presenting morphological and functional features similar to those of primary human hepatocytes).

The term "stem cell" refers to a progenitor cell capable of self-renewal, i.e., can proliferate without differentiation, whereby the progeny of a stem cell or at least part thereof substantially retains the unspecialized or relatively less specialized phenotype, the differentiation potential, and the proliferation competence of the mother stem cell. The term encompasses stem cells capable of substantially unlimited self-renewal, i.e., wherein the capacity of the progeny or part thereof for further proliferation is not substantially reduced compared to the mother cell, as well as stem cells which display limited self-renewal, i.e., wherein the capacity of the progeny or part thereof for further proliferation is demonstrably reduced compared to the mother cell.

Based on the ability to give rise to diverse cell types, a progenitor or stem cell may be usually described as totipotent, pluripotent, multipotent or unipotent. A single "totipotent" cell is defined as being capable of growing, i.e. developing, into an entire organism. A "pluripotent" cell is not able of growing into an entire organism, but is capable of giving rise to cell types originating from all three germ layers, i.e., mesoderm, endoderm, and ectoderm, and may be capable of giving rise to all cell types of an organism. A "multipotent" cell is capable of giving rise to at least one cell type from each of two or more different organs or tissues of an organism, wherein the said cell types may originate from the same or from different germ layers, but is not capable of giving rise to all cell types of an organism. A "unipotent" cell is capable of differentiating to cells of only one cell lineage.
The liver or part thereof is obtained from a "subject", "donor subject" or "donor", interchangeably referring to a vertebrate animal, preferably a mammal, more preferably a human.

As used herein, the term "isolated cell" refers generally to a cell that is not associated with one or more cells or one or more cellular components with which the cell is associated in vivo. For example, an isolated cell may have been removed from its native environment, or may result from propagation, e.g., ex vivo propagation, of a cell that has been removed from its native environment.

The term "liver" refers to the liver organ. The term "part of liver" generally refers to a tissue sample derived from any part of the liver organ, without any limitation as to the quantity of the said part or the region of the liver organ where it originates. Preferably, all cell types present in the liver organ may also be represented in the said part of liver. Quantity of the part of liver may at least in part follow from practical considerations to the need to obtain enough primary liver cells for reasonably practicing the method of the invention. Hence, a part of liver may represent a percentage of the liver organ (e.g. at least 1 %, 10 %, 20 %, 50 %, 70 %, 90 % or more, typically w/w). In other non-limiting examples, a part of liver may be defined by weight (e.g. at least 1 g, 10 g, 100 g, 250 g, 500 g, or more). For example, a part of liver may be a liver lobe, e.g., the right lobe or left lobe, or any segment or tissue sample comprising a large number of cells that is resected during split liver operation or in a liver biopsy.

The term "adult liver" refers to the liver of subjects that are post-natal, i.e. any time after birth, preferably full term, and may be, e.g., at least at least 1 day, 1 week, 1 month or more than 1 month of age after birth, or at least 1, 5, 10 years or more. Hence, an "adult liver", or mature liver, may be found in human subjects who would otherwise be described in the conventional terms of "infant", "child", "adolescent", or "adult". A skilled person will appreciate that the liver may attain substantial developmental maturity in different time postnatal intervals in different animal species, and can properly construe the term "adult liver" with reference to each species.

The term "hepatocyte" encompasses epithelial and parenchymal liver cells, including but not limited to hepatocytes of different sizes or ploidy (e.g., diploid, tetraploid, octoploid).

The term "infection" refers to the invasion of an organism's body tissues by disease causing agents, their multiplication, and the reaction of host tissues to the infectious agents and toxins they produce.

The term "treatment" refers to both therapeutic treatment and prophylactic or preventative measures, wherein the object is to prevent or slow down (lessen) the targeted pathologic condition or disorder. Those in need of treatment include those already with the disorder as well as those prone to have the disorder or those in whom the disorder is to be prevented.

The term "symptomatic treatment" refers to a therapy or treatment that only affects the symptoms but not the cause of a disease. In the case of an infection, said symptomatic treatment may be used to treat the effects caused by the infection, rather than battle the infection itself.

The term "allogeneic" as used herein means that the donated material comes from different, although often related, individual than the recipient. Allogeneic stem cell transplantation refers to a procedure in which a person receives stem cells from a genetically similar, but not identical, donor.

The term "pulmonary disease" as used herein, refers to any lung disease which is any problem in the lungs that prevents the lungs from working properly and/or causes lung damage.

The term "systemic inflammation" is to be understood as a generalized pathologic process, which can be clinically manifested in various forms, including systemic inflammatory response syndrome (SIRS), sepsis, severe sepsis, septic shock, and multiple-organ dysfunction and failure.

The term "sepsis" as used herein means a life-threatening organ dysfunction caused by a dysregulated host response to infection. Sepsis is characterized by disseminated inflammatory response elicited by microbial infections. Sepsis commonly leads to a state of immunosuppression characterized by lymphocyte apoptosis and susceptibility to nosocomial infections.

The term "sepsis-induced" as used herein, refers to all diseases and conditions that occur in a tissue, organ and/or organ system, which are the consequence of sepsis and related events. Such conditions may include, but are not limited to sepsis-induced cardiomyopathy, sepsis-induced coagulopathy, poor organ function, acute kidney injury, lung sepsis, poor organ function and/or insufficient blood flow, and the like.

The term "sepsis induced lung injury" as used herein, means acute lung injury or disorder, which occurs secondary to sepsis. The lung is the organ most often affected by the sepsis related systemic inflammatory response, resulting in acute lung injury or the more severe acute respiratory distress syndrome.

The term "adult human liver-derived progenitor cells" is used synonymously with "human adult liver-derived progenitor cells" or "Human allogeneic liver progenitor cells" or "Human allogeneic liver-derived progenitor cells"; "heterologous human adult liver-derived progenitor cells", abbreviated as "HHALPC" or "HHALPCs" or "HALPC" or "HALPCs" represent a specific type of adult human liver-derived progenitor cells, obtainable as described herein-above. A person skilled in the relevant technical field will understands that these cells have been commonly labelled as "heterologous", even though derived from human livers.

Therefore, these cells could also be labelled as "allogeneic" rather than "heterologous", to convey the connotation that they are derived from different individuals of the same species as those who will receive the cells for treatment.

The term "in vitro" as used herein denotes outside, or external to, animal or human body. The term "in vitro" as used herein should be understood to include "ex vivo". The term "ex vivo" typically refers to tissues or cells removed from an animal or human body and maintained or propagated outside the body, e.g., in a culture vessel.

The term "pharmaceutically acceptable carrier" as used herein, refers to a carrier or a diluent that does not cause significant irritation to a subject and does not abrogate the biological activity and properties of the administered composition. Examples, without limitations, of carriers are propylene glycol, saline, emulsions and mixtures of organic solvents with water.

The term "sufficient amount" means an amount sufficient to produce a desired and measurable effect, e.g., an amount sufficient to alter a protein expression profile.

The term "therapeutically effective amount" is an amount that is effective to ameliorate a symptom of a disease. A therapeutically effective amount can be a "prophylactically effective amount" as prophylaxis can be considered therapy.

The term "fibrosis" as used herein refers to the formation of excess fibrous connective tissue in an organ or tissue in a reparative or reactive process.

The term "pulmonary fibrosis" refers to a condition in which normal lung parenchyma is gradually exchanged with fibrotic tissue. The replacement of normal lung with scar tissue causes irreversible decrease in oxygen diffusion capacity.

The term "cell medium" or "cell culture medium" or "medium" refers to an aqueous liquid or gelatinous substance comprising nutrients which can be used for maintenance or growth of cells. Cell culture medium can contain serum or can be serum-free.

### DETAILED DESCRIPTION

In a first aspect, the current invention pertains to human allogeneic liver-derived progenitor cells (HALPCs), a composition comprising a therapeutically effective amount of said human allogeneic liver-derived progenitor cells or conditioned medium obtainable by culturing said cells in a cell medium for use in the (symptomatic) treatment of inflammatory lung diseases or infectious lung diseases and/or systemic inflammation in a subject.

For the purpose of the current invention, the term "pulmonary illness" or "lung disease" may apply to (inflammatory) conditions of the lung and respiratory tract, respiratory failure, pneumonitis, pneumonia, acute respiratory distress syndrome (ARDS) such as ARDStype 1 and 2 respiratory failure, interstitial lung disease (ILD), diffuse parenchymal lung disease (DPLD), idiopathic non-specific interstitial pneumonia (NSIP), Chronic obstructive pulmonary disease (COPD) and/or pulmonary fibrosis.

Clinically, acute lung injury and inflammation is seen in pneumonia and acute respiratory distress syndrome (ARDS), whereas chronic inflammation is represented by asthma and chronic obstructive pulmonary diseases (COPD). The repair of the airway epithelium after acute or chronic injury is modulated by matrix metalloproteinases (MMPs), cytokines, and growth factors produced by epithelial cells, lung-resident immune cells, fibroblasts, and chondrocytes. Inappropriate immune responses and/or aberrant repair process causes irreversible damage in lung tissue and most usually results in the development of fibrosis followed by decline in lung function. Inhaled corticosteroids are effective in patients with lung diseases, but their long-term use is associated with an increased risk of pneumonia, oral candidiasis, osteoporosis, skin bruising, and tuberculosis. Accordingly, new therapeutic agents that attenuate ongoing inflammation and prevent accumulation of fibrous connective tissue on one side and, at the same time, promote regeneration of injured alveolar epithelial cells are needed.

For the purpose of the current invention, the term "inflammatory lung diseases" or "inflammatory respiratory illness" or "inflammatory pulmonary illness" is to be understood as an illness of the lungs and respiratory tract, typically defined by inflammation of the lung tissue and/or surrounding tissue. These can be caused by airborne irritants, hazardous pollutants, toxins, allergies, by drug use (eg when treating cancer) or other medical treatments such as radiation.

The term "infectious lung disease" is typically understood as a lung disease or pathology that is caused by infection of the lung and/or the respiratory tract.

In an embodiment, said HALPCs or conditioned medium thereof are used to treat, reduce or prevent said lung disease, whether inflammatory or infectious. In a particular embodiment, said HALPCs or conditioned medium thereof are used to treat, reduce or prevent pneumonia or lung fibrosis.

In another aspect, the current invention equally pertains to the use of said human allogeneic liver-derived progenitor cells (HALPCs), a composition comprising a therapeutically effective amount of said human allogeneic liver-derived progenitor cells or conditioned medium obtainable by culturing said cells in a cell medium for use in reducing or preventing gastro-intestinal damage and/or symptoms caused by an infection in a subject. In the context of the current invention, said "gastro-intestinal damage" or "symptoms" refers to pathologies of the gastrointestinal tract, in the current case linked to the presence of an infection. Said damage may comprise but is not limited to gastroduodenal or stomach ulcers, tissue erosion, gastric and small bowel damage, liver damage, liver tissue erosion etc.

The inventors of the current invention have found that said HALPCs, compositions comprising a therapeutically effective amount of said HALPCs or conditioned medium obtained by culturing said HALPCs in a suited cell medium are particularly effective in the (symptomatic) treatment of the pathologies mentioned above. Said cells as well as conditioned medium, when being administered to a patient, are shown to reside both in lungs as in the gastrointestinal tract such as the liver, where they can act as a treatment or supportive treatment for patients suffering from inflammatory lung disease, gastrointestinal damage or symptoms by infection, or both. Due to their capacity to suppress detrimental immune responses, the cells or the medium represent new players in cell-based therapy of these pathologies.

In an embodiment of the current invention, said lung disease is an illness caused by an infectious disease. In an embodiment, said infection may be of bacterial, fungal, parasitic or viral origin. In a preferred embodiment, said infection is an infection causing respiratory problems or is a respiratory tract infection. In a further preferred embodiment, said infection is a viral infection, more preferably an infection caused by Herpesviridae, more preferably herpes simplex virus (HSV) or cytomegalovirus (CMV); Paramoxyviridae, influenza viruses, or coronaviruses. In a further embodiment, said infection is HSV, CMV, measle virus, influenza virus A, group 1 (H1N1), group 2 (H2N2), group 3 (H3N2), group 5 (H5N1, H5N2, H5N8) or group 7 (H7N7, H7N9); influenza B; influenza C; SARS-CoV-1; MERS-CoV; or SARS-CoV-2 (previously known by the provisional name 2019 novel coronavirus (2019-nCoV) . In a preferred embodiment, said infection is COVID-19 that is caused by SARS-CoV-2.

These viral infections are known to be able to cause severe pulmonary illness, especially but not exclusively in patients that are immunodeficient or suffer from pre-existing conditions. In addition, many of these infections, when not treated well, or when treatment is unavailable, may lead to systemic inflammation of other parts of the body, which may result in death.

Infection of the lung by viruses such as SARS-CoV-2 but also others may lead to a cytokine storm with elevated levels of multiple proinflammatory cytokines, which causes edema, air exchange dysfunction, acute respiratory distress, secondary infection, and which may ultimately result in death. The HALPCs as described herein as well as their conditioned medium were also found to aid in the treatment of inflammatory conditions of the lung and respiratory tract as well as systemic inflammation caused by said infection. Systemic inflammation is a generalized pathologic process, which can be clinically manifested in various forms, including systemic inflammatory response syndrome (SIRS), sepsis, severe sepsis, septic shock, and multiple-organ dysfunction and failure. It is believed that said cells inhibit the overactivation of the immune system by improving the lung microenvironment. While not wishing to be bound to theory, it is believed that said cells may lack the expression receptors (or only have low expression) typically used by pathogens to enter host cells and as such remain free of the infectious pathogen. This may aid in the recovery of the patients that exhibit severe lung disease or systemic inflammation. SARS-CoV-2 host cell infection for instance, as is also the case for SARS-CoV-1, is believed to include the recognition of angiotensin I converting enzyme 2 receptor (ACE2) by one of the spike proteins on the SARS-CoV-2 surface as well as priming of its spike protein by the cellular transmembrane protease, serine 2 (TMPRSS2). Mortality caused by COVID-19 infection relates to inflammatory lung condition called acute respiratory distress syndrome (ARDS) and to inflammatory cytokine storm. The HALPCs of the current invention are shown to have no or very low level of ACE2 and TMPRSS2 (on RNA level) and therefore remain free from infection. Their unaffected presence in the lung but also in other organs that may be affected by the virus could therefore thus reduce the further spreading of the virus.

In addition, the function of the HALPCs appear also to elicit a robust anti-inflammatory activity and therefore may counteract the action of pro-inflammatory cytokines or even reduce the concentration of the latter.

In an embodiment, the mode of administration of the HALPCs, compositions comprising a therapeutically effective amount of HALPCs or conditioned medium will be by way of intravenous, intramuscular or intraperitoneal injection, systemic transfusion, inhalation, bronchoscopic or intra-nasal instillation. In some embodiments, it may also be satisfactory to implant the HALPCs (with or without a supporting substrate such as an implantable gel or solid scaffold material) directly into the lung at or near to a site of lung injury (eg sites of inflammation and/or fibrosis). In a preferred embodiment, said cells, composition or conditioned medium will be administered intravenously by infusion. For that purpose, the cells will be formulated as a liquid, preferably a sterile liquid. For that purpose, said cells may be combined with a liquid carrier (e.g. cell culture medium or buffer) that is appropriate for the desired method of treatment, the selected route of administration, and/or storage, as well as in the preferred means for providing such pharmaceutical compositions (e.g. within a kit). The carrier can be a pharmaceutically acceptable solvent or dispersing medium containing, for example, water, saline, phosphate buffered saline, polyol (for example, glycerol, propylene glycol, liquid polyethylene glycol, and the like) and suitable mixtures thereof. Other agents of biological (e.g. antibodies or growth factor) or chemical origin (e.g. drugs, preserving or labeling compounds) that may provide any other useful effect can be also combined in such compositions.

For example, the cells may be provided as a cell suspension in any preservation medium after isolation procedure or after thawing following cryopreservation. As an example, the cell suspension may be prepared with a sterile diluent, such as a sterile aqueous solution, optionally comprising excipients such as pH-modifiers and/or human serum albumin, which is physiologically compatible with the patient.

Sterile injectable solutions can be prepared by incorporating the cells utilized in practicing the present invention in the required amount of the appropriate solvent with various amounts of the other ingredients, as desired. Such compositions may further be in admixture with a suitable carrier, diluent, or excipient such as sterile water, physiological saline, glucose, dextrose, or the like. The compositions can contain auxiliary substances such as wetting or emulsifying agents, pH buffering agents, gelling or viscosity enhancing additives, preservatives, flavoring agents, colors, and the like, depending upon the route of administration and the preparation desired.

In another or further embodiment the composition of the invention can be provided as a suspension of cells, a sponge or other three-dimensional structure where cells can grow and differentiate in vitro and/or in vivo including bioartificial devices, natural or synthetic matrices, or other systems allowing the engraftment and functionality of cells in appropriate locations (including areas of inflammation or tissue injury that expressing chemokines that help the homing and the engraftment of the cells). In particular, the HALPCs or (pharmaceutical) composition comprising said HALPCs can be administered via injection (encompassing also catheter administration, intravenously or intra-arterially) or implantation, e.g. localized injection, systemic injection, intrasplenic injection, intra-articular injection, intraperitoneal injection, intraportal injection, parenteral administration, or intrauterine injection into an embryo or fetus.

Other agents of biological (e.g. antibodies or growth factor) or chemical origin (e.g. drugs, preserving or labeling compounds) that may provide any other useful effect can be also combined in such compositions.

In an embodiment, said composition comprising the HALPCs may comprise at least 10³, 10⁶, 10⁹ or more cells (for example, between 5 million and 500 million or between 5 million and 250 million or between 50 million and 500 million or between 50 million and 250 million or between 100 million and 500 million or between 100 million and 250 million of cells for each dose or administration).
According to another or further embodiment, the dose administered to the patient is from about 50 to about 200 million HALPCs, or from about 50 to about 150 million HALPCs, such as about 100 million HALPCs. Optionally, the administration or the therapeutic use of the HALPCs or composition may comprise the administration or use of another product (which may be, for example a drug, a therapeutic agent, another cell type, or other biological material). HALPCs may be used in (or for use in) a method of treatment as described herein, wherein the patient is also administered such another product as part of the method. The other product may be administered in combination with the HALPCs, for example as part of the same composition, or separately, simultaneously or sequentially (in any order). The other product may have effects that are compatible, or even synergistic, with the effects (in particular with the therapeutic effects) of HALPCs.

In an embodiment, said HALPCs or a composition comprising a therapeutically effective amount of said adult derived human liver progenitor cells are administered to a subject in need thereof in a dose of 0.1 to 2.5 million HALPC cells per kg body weight of said subject, more preferably 0.25 to 2.5 million HALPC cells per kg body weight of said subject. In another preferred embodiment, the dose is in the range from about 0.25 to about 1.5 million cells per kg, in the range from about 0.25 to about 1.25 million cells per kg, or about 0.5 to about 1.2 million cells per kg, for example about 0.5, 0.6, 0.64 to 0.8, 1.0, or 1.2 million cells per kg, respectively. In a preferred embodiment, said dose comprises 0.5 to 1 million HALPC cells per kg body weight of said subject.
In a further or other embodiment, said HALPCs or composition is administered to said subject in the form of a liquid comprising the cells at a concentration of 0.5 to 5 million cells per mL.

In some embodiments, these amounts or doses are determined using the cell counting methods as described further below.

In any of the preceding embodiments, the composition comprising the HALPCs may be administered to the patient in the form of a sterile liquid.

In one embodiment the cells, the composition or the conditioned medium is administered via intravenous infusion, optionally using a peripheral catheter. Alternatively, the composition may be administered to the patient through a central line. The volume of the composition which is administered per infusion to a patient is preferably adapted in accordance with the patient's body weight. In one embodiment, the volume of the composition administered per infusion after final adjustment of the cell concentration may be in the range from about 5 to about 500 mL, and preferably from about 10 to about 200 mL, or from about 20 to about 150 mL, respectively.

The volume and concentration of the administered composition in the form of a sterile liquid comprising the HALPC cells or in the form of a conditioned cell medium is preferably adapted for intravenous infusion. In an embodiment, the composition may be administered to the patient in the form of a sterile liquid comprising, after final adjustment, the HALPC cells at a concentration of up to about 10 million cells per mL, and particularly of 0.5 to 5 million cells per mL. In another embodiment, the patient may be administered a sterile liquid composition with concentration of 0.5 to 2 million HALPC cells per mL, such as about 1 million HALPCs per mL. Alternatively, the cell concentration of the composition may be in the range from about 1 to about 5 million cells per mL, or from about 2 to 5 million cells per mL, respectively. These final cell concentrations may be obtained by appropriately diluting a more concentrated HALPC composition.

The volume of the composition which is administered per infusion to a patient is preferably adapted in accordance with the patient's body weight. In one embodiment, the volume of the composition administered per infusion after final adjustment of the cell concentration may be in the range from about 5 to about 500 mL, and preferably from about 10 to about 200 mL, or from about 20 to about 150 mL, respectively.

In yet another embodiment, the composition used for carrying out the invention is a sterile liquid composition which is intravenously infused to the patient at an infusion rate of about 0.1 to about 5 mL per minute, or at a rate of about 0.5 to 2 mL per minute, respectively. It is also preferred that the infusion rate is selected such that an overall infusion time of not more than about 4 hours, or even not more than about one hour, is required for administering a single dose. For example, the composition may be intravenously infused to the patient at an infusion rate of about 1 mL per minute. Further preferred is an infusion rate of about 1 to 2 mL per minute, such as about 1.5 mL per minute. In a further preferred embodiment, the infusion rate is selected in the range of about 0.5 to about 10 million cells per minute, or from about 1 to about 7.5 million cells per minute, respectively.

The composition may be administered to the patient using an infusion bag, such as a 150 mL mixing and infusion bag made of ethylenevinylacetate (EVA), e.g. MIB150 (by Hegewald or Hemedis) which holds the composition having its final concentration. The infusion bag may be connected to a tube, such as a blood administration tubing and filter set (filter pore size about 200 µm) and a flow regulator.

In another preferred embodiment, the composition is administered using a syringe pump. An example of a suitable device is the CA-700 ambulatory syringe pump (Canafusion Technology Inc.). However, any other syringe pump compatible with a syringe having the desired internal volume capacity (e.g. 50 mL) and an adjustable flow rate may also be used. The syringe pump should preferably be mounted such that the syringe has a vertical orientation. The inventors have found that, at the preferred infusion rates, the vertical orientation leads to a more homogeneous delivery of the HALPCs to the patient over the infusion time and makes agitation of the composition during the infusion process unnecessary. Accordingly, in a preferred embodiment, the composition is administered to a patient with a vertically mounted infusion pump at a rate of about 0.1 to about 5 mL per minute, or at a rate of about 0.5 to 2 mL per minute, such as 1.5 mL per minute.

The treatment might be a single dose of cells or conditioned medium or where needed, comprise of multiple doses. In a particular embodiment, said treatment is achieved by a dosing regimen comprising at least two dosings of the HALPCs or conditioned medium. In particular, a first and a second amount of cells or medium are administered to said subject in need thereof. Said second amount may be administered in a period of 1 to 60 days after the first amount, more preferably between 2 and 30 days after the first amount, or 2 to 21 days after the first amount, more preferably 2 to 5 days after the first amount.

In further embodiment, said second amount is administered 6 to 8 days, preferably 7 days after the first amount.

The dose of the first and second amount may be the same or substantially the same. In another embodiment, said dose of first en second amount may differ. For instance the dose of the first amount may comprise more cells or be administered at a higher dose than the dose of the second amount. In another embodiment, said dose of the first amount may comprise less cells or be administered at a lower dose than the dose of the second amount. Alternatively, the first amount of the composition administered to the patient may be independently selected from the second amount of the composition.

In an embodiment, said second amount comprises a second dose of 0.1 to 2.5 million HALPC cells per kg body weight of said subject, preferably 0.25 to 2.5 million HALPC cells per kg body weight, more preferably 0.25 to 1.5 million HLAPC cells per kg body weight. In another embodiment, the second amount of the composition administered to the patient comprises a dose of 1 to 2.5 million HLAPC cells per kg body weight. In another preferred embodiment, the dose is in the range from about 0.25 to about 1.5 million cells per kg, in the range from about 0.25 to about 1.25 million cells per kg, or about 0.5 to about 1.2 million cells per kg, for example about 0.5, 0.6, 0.64 to 0.8, 1.0, or 1.2 million cells per kg, respectively. In a preferred embodiment, said dose comprises 0.5 to 1 million HALPC cells per kg body weight of said subject.
In another or further embodiment, said treatment may comprise of more than two doses if deemed needed. The third and further amount may be adapted according to the needs of the patient.

In one embodiment, the HALPCs for use according to the current invention are positive for at least one mesenchymal marker. Mesenchymal markers include but are not limited to Vimentin, CD13, CD90, CD73, CD44, CD29, α-smooth muscle actin (ASMA) and CD140-b. In addition, the liver progenitor cells may secrete HGF and/or PGE2. Moreover, they can optionally be positive for at least one hepatic marker and/or exhibit at least one liver-specific activity. For example, hepatic markers include but are not limited to HNF-3B, HNF-4, CYP1A2, CYP2C9, CYP2E1, CYP3A4 and alpha-1 antitrypsin and may also include albumin (ALB). Liver-specific activities may include but are not limited to urea secretion, bilirubin conjugation, alpha-1-antitrypsin secretion and CYP3A4 activity. In a further embodiment said cells are negative for HLA-DR.

In another embodiment of the present invention, the HALPCs comprised in the composition express at least one mesenchymal marker selected from CD90, CD44, CD73, CD13, CD140b, CD29, vimentin and α-smooth muscle actin (ASMA), and secrete HGF. In a further embodiment said cells are negative for HLA-DR.
In another embodiment of the present invention, the HALPCs comprised in the composition express at least one mesenchymal marker selected from CD90, CD44, CD73, CD13, CD140b, CD29, vimentin and α-smooth muscle actin (ASMA), and secrete HGF and PGE2. In a further embodiment said cells are negative for HLA-DR. In another embodiment of the present invention, the HALPCs express at least one mesenchymal marker selected from CD90, CD44, CD73, CD13, CD140b, CD29, vimentin and α-smooth muscle actin (ASMA), and they optionally also express at least one hepatic marker and/or exhibit a liver-specific activity. In a further embodiment said cells are negative for HLA-DR.

In another embodiment of the present invention, the HALPCs coexpress at least one mesenchymal marker as described above with respect to step (c); or the marker may be selected from CD90, CD44, CD73, CD13, CD140b, CD29, vimentin and alpha-smooth muscle actin (ASMA) with one or more hepatic markers selected from alphafetoprotein (AFP), alpha-1 antitrypsin, HNF-4 and MRP2 transporter and optionally the hepatic marker albumin (sometimes also referred to as a hepatocyte marker). They optionally also exhibit a liver-specific activity selected from urea secretion, bilirubin conjugation, alpha-l-antitrypsin secretion and CYP3A4 activity. Moreover, the HALPCs preferably express HGF and PGE-2. In a further embodiment said cells are negative for HLA-DR.

In another or further embodiment of the present invention, the HALPCs are measured:
(a) positive for α-smooth muscle actin (ASMA), CD140b and optionally albumin (ALB);
(b) negative for Cytokeratin-19 (CK-19)
(c) and optionally negative for Sushi domain containing protein 2 (SUSD2).
In a further embodiment said cells are negative for HLA-DR.

In a further embodiment, the HALPCs are negative for certain markers, such as CD133, CD45, CK19 and/or CD31.

The biological activities, the markers, and the morphological/functional features listed above can be present in HALPCs in different combinations of markers, such as:
(a) positive for α-smooth muscle actin, vimentin, CD90, CD73, CD44, CD29, CD140b, and optionally albumin; and
(b) negative for Cytokeratin-19, and CD271.

The HALPCs or the compositions according to the present invention may be prepared by means of the following method:
(a) Disassociating adult liver or a part thereof to form a population of primary liver cells;
(b) Generating preparations of the primary liver cells of (a);
(c) Culturing the cells comprised in the preparations of (b) onto a support which allows adherence and growth of cells thereto and the emergence of a population of cells;
(d) Passaging the cells of (c) at least once;
(e) Isolating the cell population that is obtained after passaging of (d) that are positive for the markers identified in any one of the embodiments described herein.

Concerning Step (a) of the method, the dissociation step involves obtaining a liver or a part thereof that comprises, together with fully differentiated hepatocytes, an amount of primary cells that can be used for producing liver progenitor or stem cells.

A part of a liver can be a tissue sample derived from any part of the liver and may comprise different cell types present in the liver. The term "liver" refers to liver organ. The term "part of liver" generally refers to a tissue sample derived from any part of the liver organ, without any limitation as to the quantity of the said part or the region of the liver organ where it originates. Preferably, all cell types present in the liver organ may also be represented in the said part of liver. The quantity of the part of liver may at least in part follow from practical considerations to the need to obtain enough primary liver cells for reasonably practicing the method of the invention. Hence, a part of liver may represent a percentage of the liver organ (e.g. at least 1%, 10%, 20%, 50%, 70%, 90% or more, typically w/w). In other non-limiting examples, a part of liver may be defined by weight (e.g. at least 1 g, 10 g, 100 g, 250 g, 500 g, or more). For example, a part of liver may be a liver lobe, e.g., the right lobe or left lobe, or any segment or tissue sample comprising a large number of cells that is resected during split liver operation or in a liver biopsy.
The cells according to the invention are preferably isolated from mammalian liver or part of a liver, where the term "mammalian" refers to any animal classified as a mammal, including, but not limited to, humans, domestic and farm animals, zoo animals, sport animals, pet animals, companion animals and experimental animals, such as, for example, mice, rats, rabbits, dogs, cats, cows, horses, pigs and primates, e.g., monkeys and apes.

More preferably, the liver progenitor cell or stem cell is isolated from human liver or a part thereof, preferably human adult liver or a part thereof.

In an alternative embodiment of the invention, the adult liver or part thereof may be from a non-human animal subject, preferably a non-human mammal subject. Progenitor or stem cells or cell lines, or progeny thereof, derived as described herein from livers of non-human animal or non-human mammal subjects can be advantageously used. By means of example and not limitation, particularly suitable non-human mammal cells for use in human therapy may originate from pigs.

A donor subject may be living or dead, as determined by art-accepted criteria, such as, for example, the "heart-lung" criteria (usually involving an irreversible cessation of circulatory and respiratory functions) or the "brain death" criteria (usually involving an irreversible cessation of all functions of the entire brain, including the brainstem). Harvesting may involve procedures known in the art, such as, for example, biopsy, resection or excision.

A skilled person will appreciate that at least some aspects of harvesting liver or part thereof from donor subjects may be subject to respective legal and ethical norms. By means of example and not limitation, harvesting of liver tissue from a living human donor may need to be compatible with sustenance of further life of the donor. Accordingly, only a part of liver may typically be removed from a living human donor, e.g., using biopsy or resection, such that an adequate level of physiological liver functions is maintained in the donor. On the other hand, harvesting of liver or part thereof from a non-human animal may, but need not be compatible with further survival of the non-human animal. For example, the non-human animal may be humanely culled after harvesting of the tissue. These and analogous considerations will be apparent to a skilled person and reflect legal and ethical standards.

Liver or part thereof may be obtained from a donor, preferably a human donor, who has sustained circulation, e.g., a beating heart, and sustained respiratory functions, e.g., breathing lungs or artificial ventilation. Subject to ethical and legal norms, the donor may need to be or need not be brain dead (e.g., removal of entire liver or portion thereof, which would not be compatible with further survival of a human donor, may be allowed in brain dead human beings). Harvesting of liver or part thereof from such donors is advantageous, since the tissue does not suffer substantial anoxia (lack of oxygenation), which usually results from ischemia (cessation of circulation).

Alternatively, liver or part thereof may be obtained from a donor, preferably a human donor, who at the time of harvesting the tissue has ceased circulation, e.g., has a non-beating heart, and/or has ceased respiratory functions, e.g., has non-breathing lungs and no artificial ventilation. While liver or part thereof from these donors may have suffered at least some degree of anoxia, viable progenitor or stem cells can also be isolated from such tissues. Liver or part thereof may be harvested within about 24h after the donor's circulation (e.g., heart-beat) ceased, e.g., within about 20h, e.g., within about 16h, more preferably within about 12h, e.g., within about 8h, even more preferably within about 6h, e.g., within about 5h, within about 4h or within about 3h, yet more preferably within about 2h, and most preferably within about 1h, such as, within about 45, 30, or 15 minutes after the donor's circulation (e.g., heart-beat) ceased.

The harvested tissues may be cooled to about room temperature, or to a temperature lower than room temperature, but usually freezing of the tissue or parts thereof is avoided, especially where such freezing would result in nucleation or ice crystal growth. For example, the tissue may be kept at any temperature between about 1°C and room temperature, between about 2°C and room temperature, between about 3°C and room temperature or between about 4°C and room temperature, and may be advantageously be kept at about 4°C. The tissue may also be kept "on ice" as known in the art. The tissue may be cooled for all or part of the ischemic time, i.e., the time after cessation of circulation in the donor. That is, the tissue can be subjected to warm ischemia, cold ischemia, or a combination of warm and cold ischemia. The harvested tissue may be so kept for, e.g., up to 48h before processing, preferably for less than 24h, e.g., less than 16h, more preferably for less than 12h, e.g., less than 10h, less than 6h, less than 3h, less than 2h or less than 1h.

The harvested tissue may advantageously be but need not be kept in, e.g., completely or at least partly submerged in, a suitable medium and/or may be but need not be perfused with the suitable medium, before further processing of the tissue. A skilled person is able to select a suitable medium which can support the survival of the cells of the tissue during the period before processing.

Isolation of progenitor cells or stem cells from a liver or part of a liver is performed according to methods known in the art, for example as described in EP1969118, EP3039123, EP3140393 or WO2017149059 (see Example 1).

A population of liver primary cells is first obtained from disassociating of liver or part thereof, to form a population of primary cells from said liver or part thereof. The term "disassociating" as used herein refers to partly or completely disrupting the cellular organization of a tissue or organ, i.e. partly or completely disrupting the association between cells and cellular components of a tissue or organ to obtain a suspension of cells (a cell population) from the said tissue or organ. The suspension may comprise solitary or single cells, as well as cells physically attached to form clusters or clumps of two or more cells. Disassociating preferably does not cause or causes as small as possible reduction in cell viability. A suitable method for disassociating liver or part thereof to obtain a population (suspension) of primary cells therefrom may be any method well known in the art, including but not limited to, enzymatic digestion, mechanical separation, filtration, centrifugation and combinations thereof. In particular, the method for disassociating liver or part thereof may comprise enzymatic digestion of the liver tissue to release liver cells and/or mechanical disruption or separation of the liver tissue to release liver cells. Small, thin fragments of liver tissues that are obtained by a liver biopsy may be used directly for pursuing cell culture according to the following Step (c) without enzymatic or mechanical disruption.

Methods for disassociating liver or part thereof as above are documented in the literature as the widely used collagenase perfusion technique in two or more steps, which has been variously adapted and modified for performing it with whole livers or segments of liver. The liver tissue is perfused with a divalent cation-free buffer solution, preheated at 37°C, containing a cation-chelating agent (e.g. EDTA or EGTA). Buffer solutions can comprise salt solutions (e.g. HEPES, Williams E medium) or any other balanced salt solution that can also include salts such as NaCl and KCl, among others. This leads to disruption of the desmosomal structures that hold cells together.

The tissue is then perfused with the buffer solution containing divalent cation(s), such as Ca²⁺ and Mg²⁺, and matrix-degrading enzymes that act to digest the tissue.

The primary liver cells are usually released by gentle mechanical disruption and/or pressing through filters, to mechanically complete the cell dissociation process. Such filters may have sieve sizes that allow passage of cells through about 0.1mm, 0.25mm, 0.50mm, 1mm or more. A succession of filters with progressively smaller sieve sizes may be used to gradually disassociate the tissue and release cells. The dissociated cells are rinsed with a buffer containing protease inhibitor, serum and/or plasma to inactivate collagenase and other enzymes used in the perfusion process, and then separated from the mixture by pelleting them with low speed centrifugation (e.g. at between 10 x g and 500 x g). Most of, if not all, viable cells can be pelleted, while dead cells and cell debris are substantially eliminated and subsequently are washed with ice-cold buffer solution to purify the cell suspension. The number and quality of the primary liver cells can vary depending on the quality of the tissue, the compositions of different solutions that are used, and the type and concentration of enzyme. The enzyme is frequently collagenase but also pronase, trypsin, hyaluronidase, thermolysin, and combinations thereof can be used. Collagenase may consist of a poorly purified blend of enzymes and/or exhibit protease activity, which may cause unwanted reactions affecting the quality and quantity of viable cells that can in turn be avoided by selecting enzyme preparations of sufficient purity and quality. Other methods of harvesting primary liver cells may exclude enzymatic digestion techniques and may involve perfusing liver with solutions containing sucrose followed by mechanical disruption.

Concerning step (b) of the method, the population of primary cells as defined and obtained herein by disassociating liver or part thereof may typically be heterogeneous, i.e., it may comprise cells belonging to one or more cell types belonging to any liver-constituting cell type, including progenitor or stem cells, that may have been present in liver parenchyma and/or in the liver non-parenchymal fraction. As used herein, the term "primary cell" includes cells present in a suspension of cells obtained from a tissue or organ of a subject, e.g. liver, by disassociating cells present in such explanted tissue or organ with appropriate techniques.

Exemplary liver-constituting cell types include but are not limited to hepatocytes, cholangiocytes (bile duct cells), Kupffer cells, hepatic stellate cells (Ito cells), oval cells and liver endothelial cells. The above terms have art-established meanings and are construed broadly herein as encompassing any cell type classified as such. The term "hepatocyte" encompasses epithelial and parenchymal liver cells, including but not limited to hepatocytes of different sizes or ploidy (e.g., diploid, tetraploid, octaploid). A primary cells population may comprise hepatocytes in different proportions (0.1%, 1%, 10%, or more of total cells), according to the method of disassociating liver and/or any methods for fractioning or enriching the initial preparation for hepatocytes and/or other cell types on the basis of physical properties (dimension, morphology), viability, cell culture conditions, or cell surface marker expression by applying any suitable techniques.

The population of primary cells as defined and obtained herein by disassociating liver (or part of it) can be used immediately for establishing cell cultures as fresh primary liver cells or, preferably, stored as cryopreserved preparations of primary liver cells using common technologies for their long-term preservation.
Concerning step (c), the preparation of liver primary cells obtained in step (b) is then cultured directly onto a fully synthetic support (e.g. plastic or any polymeric substance) or a synthetic support pre-coated with feeder cells, protein extracts, or any other material of biological origin that allow the adherence and the proliferation of similar primary cells and the emergence of a population of adult liver progenitor cells having the desired markers, such markers being identified preferably at the level of protein, by means of immunohistochemistry, flow cytometry, or other antibody based technique. Primary cells are cultured in a cell culture medium sustaining their adherence and the proliferation of and the emergence of a homogenous cell population. This step of culturing of primary liver cells as defined above leads to emergence and proliferation of liver progenitor cells in the culture and can be continued until liver progenitor or stem cells have proliferated sufficiently. For example, culturing can be continued until the cell population has achieved a certain degree of confluence (e.g., at least 50%, 70%, or at least 90% or more confluent).

As an example, the cells are cultured for at least 7 days, at least 10, or at least 12 days. In an embodiment, the cells from the primary cell population are cultured within 7 and 12 days. The term "confluence" as used herein refers to a density of cultured cells in which the cells contact one another, covering substantially all of the surfaces available for growth (i.e., fully confluent).

Liver progenitor or stem cells obtained at step (c) can be further characterized by technologies that allow detecting relevant markers already at this stage (that is, before passaging cells as indicated in step (d)), as described in EP3140393 or WO2017149059. Among the technologies used for identifying such markers and measuring them as being positive or negative, Western Blot, Flow Cytometry immunocytochemistry, and ELISA are preferred since these allow marker detection at the protein level even with the low amount of liver progenitor cells that are available at this step.

The isolation of a liver progenitor cell can then be made based on the presence or absence of positive markers, as described above.

Concerning Step (d) of the method, primary cells are cultured in a cell culture medium sustaining their adherence and the proliferation of and the emergence of a homogenous cell population that, following at least one passage, is progressively enriched for liver progenitor cells or stem cells. These liver progenitor cells can be rapidly expanded for generating sufficient cells for obtaining progeny having the desired properties, as described for example in EP3140393 or WO2017149059, with cell doubling that can be obtained within 48-72 hours and maintenance of liver progenitor cells having the desired properties for at least 2, 3, 4, 5 or more passages. The term "passage" or "passaging" is common in the art and refers to detaching and dissociating the cultured cells from the culture substrate and from each other. For sake of simplicity, the passage performed after the first time of growing the cells under adherent culture conditions is herein referred to as "first passage" (or passage 1, P1) within the method of the invention. The cells may be passaged at least one time and preferably two or more times. Each passage subsequent to passage 1 is referred to herein with a number increasing by 1, e.g., passage 2, 3, 4, 5, or P1, P2, P3, P4, P5, etc.

The isolated liver progenitor cells may be plated onto a substrate which allows adherence of cells thereto, and cultured in a medium sustaining their further proliferation, generally a liquid culture medium, which may contain serum or may be serum-free. In general, a substrate which allows adherence of cells thereto may be any substantially hydrophilic substrate. Current standard practice for growing adherent cells may involve the use of defined chemical media with or without addition of bovine, human or other animal serum. These media, that can be supplemented with appropriate mixture of organic or inorganic compounds may, besides providing nutrients and/or growth promoters, also promote the growth/adherence or the elimination/detachment of specific cell types. The added serum, besides providing nutrients and/or growth promoters, may also promote cell adhesion by coating the treated plastic surfaces with a layer of matrix to which cells can better adhere. As appreciated by those skilled in the art, the cells may be counted in order to facilitate subsequent plating of the cells at a desired density.

An inert catalyst, e.g., glass beads or powder, can facilitate clotting. Advantageously, serum can be prepared using serum-separating vessels (SST), which contain the inert catalyst to mammals.

The environment in which the cells can be plated may comprise at least a cell medium, in the methods of the invention typically a liquid medium, which supports the survival and/or growth of the isolated liver progenitor cells. The liquid culture medium may be added to the system before, together with or after the introduction of the cells thereto. The term "cell medium" or "cell culture medium" or "medium" refers to an aqueous liquid or gelatinous substance comprising nutrients which can be used for maintenance or growth of cells. Cell culture media can contain serum or be serum-free.

Typically, the medium will comprise a basal medium formulation as known in the art. Many basal media formulations can be used to culture the primary cells herein, including but not limited to Eagle's Minimum Essential Medium (MEM), Dulbecco's Modified Eagle's Medium (DMEM), alpha modified Minimum Essential Medium (alpha-MEM), Basal Medium Essential (BME), Iscove's Modified Dulbecco's Medium (IMDM), BGJb medium, F-12 Nutrient Mixture (Ham), Liebovitz L-15, DMEM/F-12, Essential Modified Eagle's Medium (EMEM), RPMI-1640, Medium 199, Waymouth's MB 752/1 or Williams Medium E, and modifications and/or combinations thereof. Compositions of the above basal media are generally known in the art and it is within the skill of one in the art to modify or modulate concentrations of media and/or media supplements as necessary for the cells cultured. A preferred basal medium formulation may be one of those available commercially such as Williams Medium E, IMDM or DMEM, which are reported to sustain in vitro culture of adult liver cells, and including a mixture of growth factors for their appropriate growth, proliferation, maintenance of desired markers and/or biological activity, or long-term storage. Another preferred medium is commercially available serum-free medium that supports the growth of liver progenitor cells, such as e.g. StemMacs™ from Miltenyi.

The term "growth factor" as used herein refers to a biologically active substance which influences proliferation, growth, differentiation, survival and/or migration of various cell types, and may effect developmental, morphological and functional changes in an organism, either alone or when modulated by other substances. A growth factor may typically act by binding, as a ligand, to a receptor (e.g., surface or intracellular receptor) present in cells. A growth factor herein may be particularly a proteinaceous entity comprising one or more polypeptide chains. The term "growth factor" encompasses the members of the fibroblast growth factor (FGF) family, bone morphogenic protein (BMP) family, platelet derived growth factor (PDGF) family, transforming growth factor beta (TGF-beta) family, nerve growth factor (NGF) family, the epidermal growth factor (EGF) family, the insulin related growth factor (IGF) family, the hepatocyte growth factor (HGF) family, the interleukin-6 (IL-6) family (e.g. oncostatin M), hematopoietic growth factors (HeGFs), the platelet-derived endothelial cell growth factor (PD-ECGF), angiopoietin, vascular endothelial growth factor (VEGF) family, or glucocorticoids. Where the method is used for human liver cells, the growth factor used in the present method may be a human or recombinant growth factor. The use of human and recombinant growth factors in the present method is preferred since such growth factors are expected to elicit a desirable effect on cellular function.

Such basal media formulations contain ingredients necessary for mammal cell development, which are known per se. By means of illustration and not limitation, these ingredients may include inorganic salts (in particular salts containing Na, K, Mg, Ca, Cl, P and possibly Cu, Fe, Se and Zn), physiological buffers (e.g., HEPES, bicarbonate), nucleotides, nucleosides and/or nucleic acid bases, ribose, deoxyribose, amino acids, vitamins, antioxidants (e.g., glutathione) and sources of carbon (e.g. glucose, pyruvate, e.g., sodium pyruvate, acetate, e.g., sodium acetate), etc. It will also be apparent that many media are available as low-glucose formulations with or without sodium pyruvate.

For use in culture, basal media can be supplied with one or more further components. For example, additional supplements can be used to supply the cells with the necessary trace elements and substances for optimal growth and expansion. Such supplements include insulin, transferrin, selenium salts, and combinations thereof. These components can be included in a salt solution such as, but not limited to, Hanks' Balanced Salt Solution (HBSS), Earle's Salt Solution. Further antioxidant supplements may be added, e.g., β-mercaptoethanol. While many basal media already contain amino acids, some amino acids may be supplemented later, e.g., L-glutamine, which is known to be less stable when in solution. A medium may be further supplied with antibiotic and/or antimycotic compounds, such as, typically, mixtures of penicillin and streptomycin, and/or other compounds, exemplified but not limited to, amphotericin, ampicillin, gentamicin, bleomycin, hygromycin, kanamycin, mitomycin, mycophenolic acid, nalidixic acid, neomycin, nystatin, paromomycin, polymyxin, puromycin, rifampicin, spectinomycin, tetracycline, tylosin, and zeocin.

Hormones can also be advantageously used in cell culture and include, but are not limited to D-aldosterone, diethylstilbestrol (DES), dexamethasone, estradiol, hydrocortisone, insulin, prolactin, progesterone, somatostatin/human growth hormone (HGH), thyrotropin, thyroxine, L-thyronine, epithelial growth factor (EGF) and hepatocyte growth factor (HGF). Liver cells can also benefit from culturing with triiodithyronine, α-tocopherol acetate, and glucagon.

Lipids and lipid carriers can also be used to supplement cell culture media. Such lipids and carriers can include, but are not limited to cyclodextrin, cholesterol, linoleic acid conjugated to albumin, linoleic acid and oleic acid conjugated to albumin, unconjugated linoleic acid, linoleic-oleic-arachidonic acid conjugated to albumin, oleic acid unconjugated and conjugated to albumin, among others. Albumin can similarly be used in fatty-acid free formulations.

Also contemplated is supplementation of cell culture medium with mammalian plasma or sera. Plasma or sera often contain cellular factors and components that are necessary for viability and expansion. The use of suitable serum replacements is also contemplated. Suitable sera or plasmas for use in the media as described herein may include human serum or plasma, or serum or plasma from non-human animals, preferably non-human mammals, such as, e.g., non-human primates (e.g., lemurs, monkeys, apes), fetal or adult bovine, horse, porcine, lamb, goat, dog, rabbit, mouse or rat serum or plasma, etc. In another embodiment, any combination of the above plasmas and/or sera may be used in the cell medium.

When passaged, the cultured cells are detached and dissociated from the culture substrate and from each other. Detachment and dissociation of the cells can be carried out as generally known in the art, e.g., by enzymatic treatment with proteolytic enzymes (e.g., chosen from trypsin, collagenase, e.g., type I, II, III or IV, dispase, pronase, papain, etc.), treatment with bivalent ion chelators (e.g., EDTA or EGTA) or mechanical treatment (e.g., repeated pipetting through a small bore pipette or pipette tip), or any combination of these treatments.

A suitable method of cell detachment and dispersion should ensure a desired degree of cell detachment and dispersion, while preserving a majority of cells in the culture. Preferably, the detachment and dissociation of the cultured cells would yield a substantial proportion of cells as single, viable cells (e.g., at least 50%, 70%, 90% of the cells or more). The remaining cells may be present in cell clusters, each containing a relatively small number of cells (e.g., on average, between 1 and 100 cells).

The so detached and dissociated cells (typically as a cell suspension in an isotonic buffer or a medium) may be re-plated onto a substrate which allows the adherence of cells thereto, and are subsequently cultured in a medium as described above sustaining the further proliferation of HALPCs and of HALPC Progeny. These cells may be then cultured by re-plating them at a density of between 10 and 105 cells/cm2, and at a splitting ratio between about 1/16 and 1/2, preferably between about 1/8 and 1/2, more preferably between about 1/4 and 1/2. The splitting ratio denotes the fraction of the passaged cells that is seeded into an empty (typically a new) culture vessel of the same surface area as the vessel from which the cells were obtained. The type of culture vessel, as well as of surface allowing cell adherence into the culture vessel and the cell culture media, can be the same as initially used and as described above, or may be different. Preferably, cells are maintained onto CellBind or any other appropriate support that is coated with extracellular matrix proteins (such as collagens, and preferably collagen type I) or synthetic peptides that are acceptable in GMP conditions.

Concerning step (e) above, the isolation of population of HALPCs applies to cells that are positive for the listed markers, further validating the criteria for initially identifying HALPCs at step (c) above but that can be more easily established given the higher amount of cells that are available after passaging.

The terms "cell population" and "population of cells" refer generally to a group of cells. Unless indicated otherwise, the term refers to a cell group consisting essentially of or comprising cells as defined herein. A cell population may consist essentially of cells having a common phenotype or may comprise at least a fraction of cells having a common phenotype. Cells are said to have a common phenotype when they are substantially similar or identical in one or more demonstrable characteristics, including but not limited to morphological appearance, the level of expression of particular cellular components or products (e.g., RNA or proteins), activity of certain biochemical pathways, proliferation capacity and/or kinetics, differentiation potential and/or response to differentiation signals or behavior during in vitro cultivation (e.g., adherence or monolayer growth). Such demonstrable characteristics may therefore define a cell population or a fraction thereof. A cell population may be "substantially homogeneous" if a substantial majority of cells have a common phenotype. A "substantially homogeneous" cell population may comprise at least 60%, e.g., at least 70%, at least 80%, at least 90%, at least 95%, or even at least 99% of cells having a common phenotype, such as the phenotype specifically referred to (e.g., the phenotype of liver progenitor or stem cells of the invention, or to progeny of liver progenitor or stem cells of the invention). Moreover, a cell population may consist essentially of cells having a common phenotype such as the phenotype of liver progenitor or stem cells of the invention (i.e. a progeny of liver progenitor or stem cells of the invention) if any other cells present in the population do not alter or have a material effect on the overall properties of the cell population and therefore it can be defined as a cell line.
Cell count, irrespective of the method which is applied, can be performed on the cell suspension at final harvest, or in the course of preparing the formulation of the pharmaceutical composition to be administered to the patient or during the quality control testing. Any method known in the art can be used, such as the Manual Count Method using Burker Chamber and the Automated Nucleocounter "NC-200". The aim of these methods is to determine the total amount of cells as well as the amount of the viable ones.

### Manual count using Burker Chamber

The manual count method using a Burker chamber is based on the Trypan Blue exclusion test.
The cell suspension is diluted with PBS to count between 100 and 200 viable cells per chamber. Trypan blue is added to cell suspension using a ratio 1:1. The cells are counted by microscopy and cell counter. The white cells are viable cells; the blue cells are dead cells. The percentage of viable and dead cells is then calculated. Two cell counts are performed. If the delta Δ between both counts is > 15%, a third count is carried out.

### Automated Nucleocounter "NC-200"

The Nucleocounter NC-200 1-step provides a high precision automatic cell counter based on fluorescent microscopy and advanced image analysis to perform image cytometric exclusion of dead cells and total cells. The one-step method uses Vial-Cassette™ containing immobilized fluorescent dyes, i.e. Acridine orange and DAPI (4',6-diamidino-2-phenylindole) that automatically stain the total and dead cell populations respectively.

The cell suspension is diluted to count between 7x10⁵ and 2x10⁶ total cells/ml. The Vial-Cassette™ will pipette a calibrated volume and the NC-200 will automatically determine the total and dead cell concentration and the percentage of viability. All counts are performed in triplicate. The reportable value is the mean out of three valid results of three independently drawn samples. To be valid, total cell count must be between and the coefficient of variation (CV) must not exceed 15.0% for the total cell concentration and for the viability.

The current invention equally relates to a conditioned medium obtainable by culturing human liver progenitor or stem cells as described above and for the uses as described above. In addition to the cells as such, also the medium that was harvested from the cell cultures, commonly known as conditioned medium, was shown to facilitate the treatment of inflammatory lung diseases, gastrointestinal damage caused by infection and/or systemic inflammation.

In a particularly preferred embodiment, said conditioned medium of the invention is cell-free. The cell-free nature can be obtained by conventional methods in the art such as filtration, enzymatic digestion, centrifugation, absorption, and/or separation by chromatography, or repetitions and/or combinations of such methods. Said conditioned medium further comprises soluble proteins, microvesicles and exosomes.

The conditioned medium obtainable by culturing the HALPCs described above was found to comprise soluble proteins, among others, growth factors, chemokines, matrix metalloproteases, and pro- and anti-inflammatory cytokines whose presence can provide useful biological activities. These components are presumed to be secreted by the cells in the medium.

In one embodiment, the isolated HALPCs are plated onto a substrate which allows adherence of cells thereto, and cultured in a medium sustaining their further proliferation, generally a liquid culture medium, which may contain serum or may be serum-free. In general, a substrate which allows adherence of cells thereto may be any substantially hydrophilic substrate. Current standard practice for growing adherent cells may involve the use of defined chemical media with or without addition of bovine, human or other animal serum. In a particularly preferred embodiment, such culturing medium comprises serum. These media, which can be supplemented with appropriate mixture of organic or inorganic compounds may, besides providing nutrients and/or growth promoters, also promote the growth/adherence or the elimination/detachment of specific cell types. The added serum, besides providing nutrients and/or growth promoters, may also promote cell adhesion by coating the treated plastic surfaces with a layer of matrix to which cells can better adhere. As appreciated by those skilled in the art, the cells may be counted in order to facilitate subsequent plating of the cells at a desired density. In another particularly preferred embodiment, such culturing medium is serum-free. A method for producing a cell-free conditioned medium as taught herein may comprise the step of obtaining HALPCs by any of the above methods, culturing HALPCs in a cell culture medium, and separating the cell culture medium from HALPCs.
The environment in which the cells are plated may comprise at least a cell medium, in the methods of the invention typically a liquid medium, which supports the survival and/or growth of the isolated liver progenitor or stem cells. The liquid culture medium may be added to the system before, together with or after the introduction of the cells thereto. Preferred media formulations have been described above.

Said method can be performed by using the cell culture medium that is a serum-free medium, by modifying specific conditions of cell culture, and/or by separating the cell culture medium from said human liver progenitor or stem cells after culturing human liver progenitor or stem cells at given time points (e.g. at least 2, 4, 6, 8, 12, 24 hours). Obtained conditioned medium have a composition enriched (or depleted) in soluble proteins, RNAs, exosomes and/or microvesicles that are either degraded (or unstable) within the conditioned media or secreted by human liver progenitor or stem cells not in regular manner but only before or after a certain number of hours (and thus not progressively accumulated in the conditioned medium). Relevant time points can be shorter (e.g. 2 hours or less) or longer such as at 24 hours, at 36 hours or more hours. By obtaining samples of said conditioned medium at these time points or at intermediate ones (such as 1, 2, 4, 6, 8, 12, or 18 hours) and testing such samples for their composition and/or activities, the optimal timing for obtaining the desired conditioned medium derived from human liver progenitor or stem cells can be determined.

In one embodiment, said conditioned medium is a product derived from cell-free conditioned medium obtainable by culturing HALPCs. Said product is a fraction obtained by fractioning said conditioned medium. Such fractioning may comprise applying one or more technologies known in the art, such as for example filtering, enzymatically digesting, centrifuging, adsorbing, and/or separating by chromatography. The term "fraction" as used herein, refers to a part, composition or derivative obtainable from the conditioned medium of the invention.

The conditioned medium and/or the fraction thereof, typically contain soluble proteins, RNAs, exosomes and/or microvesicles.

In another embodiment the conditioned medium and/or the fraction thereof contain microvesicles that are characterized and, when appropriate, selected according to their size (in certain embodiments, size smaller than 0.40 µm), molecular weight, and/or composition.

In another and further embodiment the conditioned medium and/or the fraction thereof contain exosomes that are characterized (in certain embodiments, size smaller than 80 nm) and, when appropriate, selected according to their size, molecular weight, and/or composition.

In another preferred embodiment, the conditioned medium and the fraction thereof comprise RNA, for example miRNA.

Particularly desired concentrations of such soluble proteins, RNA, exosomes and/or microvesicles conditioned medium and/or in a fraction thereof, can be obtained for example by appropriately concentrating (or diluting) the respective preparation at least about 5-fold, at least about 10-fold, at least about 20-fold, at least about 50-fold, or at least about 100-fold. Hence, certain embodiments provide so-concentrated or so-diluted conditioned medium and/or in a fraction thereof.

As mentioned, the conditioned medium and/or fraction thereof, is suitable for the use as described above. Such medical, e.g., prophylactic or therapeutic, use may involve using conditioned medium and/or fraction thereof, alone or in combination with one or more exogenous active ingredients, which may be suitably added.

Examples of such exogenous active ingredients include cells (e.g., HALPCs or other cells suitable for ex vivo or in vivo applications), proteins (e.g., matrix metalloproteases, growth factors, chemokines, cytokines, hormones, antigens, or antibodies), nutrients (e.g., sugars or vitamins) and/or chemicals (e.g., drugs with antimicrobial, anti-inflammatory, or antiviral properties) that were not initially present in conditioned medium and/or fraction thereof, and that are known to be effective as medicaments for the desired indication.

In an embodiment of the invention, a cell lysate of the cells as described above is used, rather than the actual cells. Said cell lysate may be obtained by any means known in the art, such as enzyme digestion of cells or a cell culture, detergent and/or buffer treatment, and the like.

In a further preferred embodiment, the present invention provides pharmaceutical formulations comprising a pharmaceutically effective amount of liver progenitor or stem cells, lysates thereof and/or conditioned cell medium. The pharmaceutical formulations may optionally also further comprise a pharmaceutically effective amount of one or more exogenous active ingredients, which may be of the type discussed above, e.g., the cells, proteins, nutrients, and/or chemicals. The exogenous active ingredients may be of the type discussed above, e.g., the cells, proteins, nutrients, and/or chemicals.

Accordingly, it is to be understood that the present invention extends to a method of cellular therapy for an inflammatory lung disease or condition in a subject, optionally caused by infection, comprising administering to said subject a therapeutically effective amount of HALPCs as described above, as well as to a composition thereof (ie a composition comprising HALPCs as described above, in combination with a pharmaceutically-acceptable carrier) or a conditioned medium thereof and, further, to the use of HALPCs or said conditioned medium as described above in the preparation of a composition for cellular therapy for a lung disease or condition in a subject caused by infection, wherein said composition comprises said HALPCs in combination with a pharmaceutically-acceptable carrier and/or the conditioned medium obtained from culturing said HALPCs in an appropriate cell medium. It can also be understood that the current invention provides for a method of relieving and treating widespread inflammation caused by infection, such as an influenza or coronavirus infection.

Finally, the current invention is also understood to provide HALPCs, compositions or conditioned medium thereof as described above for the use in the manufacture of a medicament for a lung disease or condition in a subject caused by infection.

The invention is further described by the following non-limiting examples which further illustrate the invention, and are not intended to, nor should they be interpreted to, limit the scope of the invention.

### EXAMPLES

### Example 1: Preparation of HALPCs

HALPCs were prepared as described in EP 3140393 or WO2017/149059 from livers of healthy cadaveric or non-heart beating donors. Briefly, liver cell preparations are re-suspended in Williams' E medium supplemented with 10% FBS, 10 mg/ml INS, 1 mM DEX. The primary cells are cultured on Corning® CellBIND® flasks and cultured at 37°C in a fully humidified atmosphere containing 5% CO₂. After 24 hours, medium is changed in order to eliminate the non-adherent cells and thereafter renewed twice a week, whereas the culture is microscopically followed every day. Culture medium is switched after 12-16 days to high glucose DMEM supplemented with 9% FBS. A cell type with mesenchymal-like morphology emerges and proliferates. When reaching 70-95% confluence, cells are trypsinized with recombinant trypsin and 1 mM EDTA and re-plated at a density of 1-10 x 10³ cells/cm². At each passage, cells were trypsinized at 80-90% confluency.

Testing of the cells confirmed that they expressed, inter alia, the following markers: CD90, CD73, Vimentin, and ASMA. The cells were also tested and found negative, or exhibit very low expression, with respect to the following markers: CD133, CD45, CK19 and CD31. The HALPCs were filled into vials in aliquots of 5 mL, each comprising 10 to 50x10⁶ cells/ml, equivalent to 50 to 250x10⁶ cells per vial, and frozen.

### Example 2: Production of conditioned medium

Conditioned medium was obtained from culturing cells as described above in suitable cell medium: at passage 4 or 5, the cells were washed and the culture medium was replaced by fresh culture medium (DMEM + 0.5 to 10 % FBS). After 6 to 96 hours, generally 24 hours incubation, supernatants were collected and cleared from floating cells and debris by means of centrifugation and stored for further assays. The concentration of secreted protein is expressed as nanograms (ng) or picograms (pg) that are secreted in 24 hours by 10⁵ cells during 24 hours.

### Example 3: Use of HALPCs in SARS-CoV-2 patients, in the context "hospital exemption" according in the frame of hospital exemption as defined bv the Belgian law

### Background

On 11th March 2020, WHO characterized SARS-CoV-2 (COVID-19) as a pandemic and highlighted the fact that they "never before seen a pandemic that can be controlled, at the same time". On 17th March 2020, 196'639 cases were confirmed worldwide with total deaths of 7'893. Based on a Chinese report, about 1 out 4 hospitalised patients are transferred into Intensive Care Unit (ICU) and 1 out 20 die. The median hospital duration stay is 10 days.

Current reports show that respiratory symptoms of COVID-19 are most common manifestations but gastro-intestinal symptoms occur early and are followed by respiratory symptoms. Given the wide distribution of cells in the body that can be infected by COVID-19, it is possible that mortality relates not only to Acute Respiratory Distress Syndrome (ARDS) but also to more widespread inflammation such as cytokine storm and organ disfunctions.

There is currently no vaccine and no virus-specific antiviral treatment is recommended. In particular, none of the various tested therapies was observed to carry benefit among patients with ARDS. It follows that there is an urgent need to find and/or develop effective therapies for patients with severe COVID-19 infection.

### Scientific rationale for using cell therapy in patients with severe SARS-CoV-2 (COVID-19) infection

Lung disfunction with gas exchange, oedema and multi-organ failure are aggravated by widespread inflammation (cytokine storm). Avoiding or reducing the cytokine storm is viewed as key for treating COVID-19 infected patients (Y. Liu et al. Clinical features of progression of acute respiratory distress syndrome in coronavirus disease 2019. Medrxiv (27 February 2020)). Cell therapies appear as promising given the strong immunomodulation effect by stem cells. Indeed, injected allogenic Mesenchymal Stem Cells (MSC) are being tested in order to modulate and reduce various severe inflammatory diseases.

A report led by Chinese investigators dated April 2020 has shown promising results with improved outcome of patients with COVID-19 pneumonia (Z. Leng et al. Transplantation of ACE2- Mesenchymal Stem Cells improve the outcome of patients with COVID-19 pneumonia. Aging and Disease 2020, 11; 216-228). This was a pilot trial using intravenous MSC transplantation into seven patients, and clinical outcomes and laboratory tests were conducted. Transplanted cells were also tested for their expression for virus receptor (ACE-2) and needed protease (TMPRSS2) and were shown to be negative for those proteins (and thus assumed to be resistant to virus infection). Clinical improvement, reduction of inflammatory factors and lack of virus receptor by transplanted cells strongly suggest that cell therapy is a promising therapeutic approach for patients with severe COVID-19 infection.

### Aim of the current trial

Because of known ability of MSC therapies to reduce the inflammation in various severe inflammatory conditions as demonstrated before COVID-19 pandemic, it is no surprise to see several companies having started or announced to launch clinical trial in the context of COVID-19 pneumonia.

While most if not all clinical tests that are public are making use of MSC to tackle the mortality by reducing the cytokine storm, the latter cells have some drawbacks.

It is therefore advantageous to evaluate therapeutic cells that have the ability to migrate both in lung and in other systems such as the gastro-intestinal system in order to further reduce inflammation and possibly mortality caused by SARS-CoV-2 (COVID-19) infection. There lies an enormous therapeutic benefit to use these cells with demonstrated anti-inflammatory action on liver which can produce pro-inflammatory factors (CRP) upon infection.

### Set up trial

Around 20 patients SARS-CoV-2 (COVID-19) infected patients with severe conditions, including severe pulmonary illness are tested in a randomised controlled trial by using fast track approval by regulatory agencies.

A composition comprising HALPCs according to one of the embodiments as described above in the detailed description, known to migrate to both lungs and liver, is used. The cells are formulated to be administered via intravenous route.

A dose ascending scheme is used, starting with a low dose of 0.25 million cells/kg, complemented two days later by a dose of 0.5 million cells /kg in the first patients. According to tolerance, the dose of 1.2 million cells/kg could be proposed, as currently used in an on-going Phase IIb clinical trial of the applicant in Acute on Chronic Liver Failure (Clinical trial HEP102 sponsored by Promethera Biosciences, registered under EudraCT 2019-003051-11). The investigators will monitor and evaluate the clinical outcome of the patients (vital signs, respiratory signs, ventilation parameters such as FiO2, frequency, pressure), and several laboratory parameters in order to generate initial signal of safety and of efficacy of the HALPCs for treating SARS-CoV-2 infection. Whenever feasible, Lung CT scan will be performed in order to document lung recovery.

The value in economic terms and common welfare for preparing the response against a future outbreak is beyond doubt. Indeed, it is now widely recognised that another coronavirus epidemy is a serious risk as COVID-19 is the 3rd outbreak by corona viruses.

It is clear that the method according to the invention, and its applications, are not limited to the presented examples.

## Claims

1. Human allogeneic liver-derived progenitor cells (HALPCs), a composition comprising a therapeutically effective amount of said human allogeneic liver-derived progenitor cells or conditioned medium obtainable by culturing said cells in a cell medium for use in the (symptomatic) treatment of inflammatory lung diseases, infectious lung diseases and/or systemic inflammation.

2. Human allogeneic liver-derived progenitor cells (HALPCs), composition comprising a therapeutically effective amount of said HALPCs or conditioned medium obtainable by culturing said cells in a cell medium, wherein said lung diseases such as lung damages and/or systemic inflammation are due to an infection.

3. Human allogeneic liver-derived progenitor cells (HALPCs), a composition comprising a therapeutically effective amount of said human allogeneic liver-derived progenitor cells or conditioned medium obtainable by culturing said cells in a cell medium for use in reducing or preventing gastro-intestinal damage and symptoms caused by an infection.

4. Human allogeneic liver-derived progenitor cells (HALPCs), a composition comprising a therapeutically effective amount of said adult derived human liver progenitor cells or conditioned medium obtainable by culturing said cells in a cell medium for use according to claim 2 or 3, wherein said infection is a viral infection.

5. Human allogeneic liver-derived progenitor cells (HALPCs), a composition comprising a therapeutically effective amount of said adult derived human liver progenitor cells or conditioned medium obtainable by culturing said cells in a cell medium for use according to claim 4 wherein said infection is a viral infection causing respiratory problems or an infection of the respiratory tract.

6. Human allogeneic liver-derived progenitor cells (HALPCs), a composition comprising a therapeutically effective amount of said adult derived human liver progenitor cells or conditioned medium obtainable by culturing said cells in a cell medium for use according to any of the previous claims wherein said infection is an infection caused by influenza viruses, Paramyxoviridae such as measles, Herpesviridae such as HPV, CMV or coronaviruses.

7. Human allogeneic liver-derived progenitor cells (HALPCs), a composition comprising a therapeutically effective amount of said adult derived human liver progenitor cells or conditioned medium obtainable by culturing said cells in a cell medium for use according to claim 6 wherein said infection is SARS-CoV-1, SARS-CoV-2 or MERS-CoV.

8. Human allogeneic liver-derived progenitor cells (HALPCs), a composition comprising a therapeutically effective amount of said adult derived human liver progenitor cells or conditioned medium obtainable by culturing said cells in a cell medium for use according to any of the previous claims, wherein said cells or composition is administered to said subject in a dose of 0.25 to 2.5 million HALPC cells per kg body weight of said subject.

9. Human allogeneic liver-derived progenitor cells (HALPCs), a composition comprising a therapeutically effective amount of said adult derived human liver progenitor cells or conditioned medium obtainable by culturing said cells in a cell medium for use according to any of the previous claims, wherein said cells or composition is administered to said subject in a dose of 0.25 to 1.5 million HALPC cells per kg body weight of said subject.

10. Human allogeneic liver-derived progenitor cells (HALPCs), a composition comprising a therapeutically effective amount of said adult derived human liver progenitor cells or conditioned medium obtainable by culturing said cells in a cell medium for use according to any of the previous claims, wherein said cells or composition is administered to said subject in the form of a liquid comprising the cells at a concentration of 0.25 to 5 million cells per mL.

11. Human allogeneic liver-derived progenitor cells (HALPCs), a composition comprising a therapeutically effective amount of said adult derived human liver progenitor cells, or conditioned medium obtainable by culturing said cells in a cell medium for use according to claim 9, wherein the liquid composition is intravenously infused to the subject, preferably at an infusion rate of 0.5 to 2 mL per minute.

12. Human allogeneic liver-derived progenitor cells (HALPCs), a composition comprising a therapeutically effective amount of said adult derived human liver progenitor cells or conditioned medium obtainable by culturing said cells in a cell medium for use according to any of the previous claims, wherein a first and a second amount of cells are administered to said subject, said second amount is administered 2 to 21 days after the first amount.

13. Human allogeneic liver-derived progenitor cells (HALPCs), a composition comprising a therapeutically effective amount of said adult derived human liver progenitor cells or conditioned medium obtainable by culturing said cells in a cell medium for use according to claim 11, wherein said second amount is administered 2 to 5 days.

14. Human allogeneic liver-derived progenitor cells (HALPCs), a composition comprising a therapeutically effective amount of said adult derived human liver progenitor cells or conditioned medium obtainable by culturing said cells in a cell medium for use according to claim 11 or 12, wherein said second amount comprises a second dose of 0.25 to 2.5 million HALPC cells per kg body weight, preferably 0.5 to 1 million HLAPC cells per kg body weight.
